# EUROPÄISCHE PATENTSCHRIFT

(11) **EP 2 166 963 B1**
(45) Veröffentlichungstag und Bekanntmachung des Hinweises auf die Patenterteilung: **08.11.2017**
(21) Anmeldenummer: 08758527.9
(22) Anmeldetag: 15.05.2008
(51) Int. Cl.: A61B 17/16, A61B 17/225, A61B 17/88, A61B 17/92, A61B 18/14, A61B 18/20, A61B 17/00, A61B 18/00, A61B 18/18, A61F 9/008, A61F 9/009, A61N 1/32, A61B 17/22

(54) **MEDIZINISCHES GERÄT ZUR BEHANDLUNG DES MENSCHLICHEN ODER TIERISCHEN KÖRPERS MIT MECHANISCHEN DRUCK- ODER STOSSWELLEN**
MEDICAL APPARATUS FOR THE TREATMENT OF A HUMAN OR ANIMAL BODY BY MEANS OF MECHANICAL PRESSURE WAVES OR SHOCK WAVES
APPAREIL MÉDICAL POUR TRAITER LE CORPS D'UN ÊTRE HUMAIN OU D'UN ANIMAL AVEC DES ONDES DE PRESSION OU DES ONDES DE CHOC MÉCANIQUES

(30) Priorität: 31.05.2007 DE 202007007920 U
(43) Veröffentlichungstag der Anmeldung: 31.03.2010
(73) Patentinhaber: STORZ MEDICAL AG, 8274 Tägerwilen (CH)
(72) Erfinder: HEINE, Gerold, 78337 Ohningen (DE); IRION,Klaus, M., 78576 Emmingen-Liptingen (DE); MARLINGHAUS, Ernst, H., 8598 Bottighofen (CH); SCHULZ, Manfred, 8274 Tägerwilen (CH); NOVAK, Pavel, 8234 Stetten (CH)
(74) Vertreter: Witte, Weller & Partner Patentanwälte mbB
(86) Internationale Anmeldenummer: PCT/EP2008/003883
(87) Internationale Veröffentlichungsnummer: WO 2008/145260

(56) Entgegenhaltungen:
- EP-A- 1 163 882
- WO-A-94/17771
- WO-A-96/33661
- DE-A1- 2 256 127
- DE-A1- 19 740 825
- DE-C1- 19 859 553
- US-A- 4 727 875
- US-A- 4 821 729

## Beschreibung

Die Erfindung betrifft ein medizinisches Gerät zur Behandlung des menschlichen oder tierischen Körpers mit mechanischen Druck- oder Stoßwellen, mit einer Vorrichtung zur Erzeugung der Druck- oder Stoßwellen, die zumindest ein Applikatorteil aufweist, das zur Erzeugung der Druck- oder Stoßwellen auf einer proximalen Seite mit Stößen beaufschlagbar ist und das auf einer distalen Seite die Druck- oder Stoßwellen in den Körper einkoppelt.

Ein solches Gerät ist aus dem Dokument DE 197 25 477 C2 bekannt.

Unter "Behandlung des menschlichen oder tierischen Körpers" ist im Sinne der vorliegenden Erfindung beispielsweise die Behandlung von Weichgewebe, insbesondere zur Schmerztherapie, die Behandlung von Knochengewebe, die Zertrümmerung von Körpersteinen, die Entfernung von Plaque in Gefäßen, die Behandlung der Zähne, aber auch das Entfernen von Knochenzement oder das Eintreiben von Knochennägeln oder -drähten zu verstehen.

Das aus dem oben genannten Dokument DE 197 25 477 C2 bekannte medizinische Gerät dient zur Behandlung bei Knochenbrüchen, Enthesiopathien, Tendopathien oder auch bei Paradonthose. Ein weiteres Einsatzgebiet dieses bekannten Geräts ist die Schmerztherapie im knochennahen Weichteilbereich des Haltungs- und Bewegungsapparates. Alle diese Anwendungen des bekannten medizinischen Geräts sind auch mit dem medizinischen Gerät der vorliegenden Erfindung durchführbar.

Das bekannte Gerät weist als Vorrichtung zur Erzeugung der Druck- oder Stoßwellen ein Applikatorteil auf, das über ein periodisch bewegliches Schlagteil mit Stößen beaufschlagt wird. Das Schlagteil wird dazu entlang einer Beschleunigungsstrecke hin und her bewegt, wobei das Schlagteil über einen Druckluftantrieb zwischen einer proximalen Ausgangsstellung und einer distalen Endstellung, in der das Schlagteil gegen das Applikatorteil prallt, bewegt wird.

Die von dem Applikatorteil erzeugten Druck- oder Stoßwellen entfalten nach Einkopplung in den Körper in dem zu behandelnden Körperareal eine mechanische Druck- oder Stoßwellenwirkung.

Das Dokument DE 198 59 553 C1 offenbart ein Therapiegerät mit einer eine akustische Achse aufweisenden Quelle zur Erzeugung von in einem auf der akustischen Achse liegenden Fokus zusammenlaufenden akustischen Wellen. Die Quelle weist Mittel zur Erzeugung von Licht auf, die einen gebündelten Lichtstrahl sichtbaren Lichtes mit wenigstens im Wesentlichen parallelem Strahlengang aussenden, der wenigstens im Wesentlichen mit der akustischen Achse der Quelle zusammenfällt und diese sichtbar macht.

Das Dokument EP 1 163 882 A1 offenbart eine Vorrichtung zum Entfernen von Körpersteinen mit einem intrakorporalen Lithotripter, die eine hohle Metallsonde bzw. Sonotrode aufweist, die für eine Steinzertrümmerung durch einen elektrisch angesteuerten Ultraschallwandler zu longitudinalen Schwingungen angeregt wird. Die Vorrichtung ist umschaltbar auf einen reversiblen Antrieb eines Schlagteils, mit dem auf einen Massekörper einer zusätzlich vorgesehenen Stoßsonde eine periodische Stoßkraft ausgeübt wird, um eine Ausbildung von Druck- bzw. Stoßwellen in der Stoßsonde zu erzeugen und damit eine alternative Möglichkeit für die Steinzertrümmerung zu erhalten.

Das Dokument US 4,821,729 offenbart eine Vorrichtung und ein Verfahren für die nicht-invasive Fragmentation von Körpersteinen. Die Vorrichtung weist neben der Quelle zur Stoßwellenerzeugung einen Ultraschallwandler zur Verifikation der Lage des Fokus der Stoßwellen auf.

Das Dokument DE 197 40 825 A1 offenbart ein Gerät zur Erzeugung röhrenförmiger Kanäle in Muskelgewebe, mit dem unabhängig voneinander die Wandlung des röhrenförmigen Kanals gezielt thermisch beeinflusst werden kann und gleichzeitig in der Umgebung der Wandlung stoßwellenartige Druckamplituden erzeugt werden können. Das Gerät weist helixartig gewundene Elektroden auf, wobei durch Anlegen von Hochspannungspulsen an die Elektroden durch Funkendurchschlag Stoß- bzw. Druckwellen erzeugt werden können.

Das Dokument WO 94/17771 A2 offenbart ein Gerät zur Schmerztherapie und/oder zur Beeinflussung des vegetativen Nervensystems. Das Gerät weist eine Druckimpulsquelle sowie eine Ultraschall-Ortungseinrichtung auf.

Das Dokument US 4,727,875 offenbart ein Gerät zur Zertrümmerung von Steinen im Körper, wobei Stoßwellen mittels eines Schlagteilantriebs erzeugt werden, und wobei eine zusätzliche Ultraschallortung im Behandlungsgebiet vorgesehen ist.

Der vorliegenden Erfindung liegt die Aufgabe zugrunde, ein Gerät der eingangs genannten Art dahingehend weiterzubilden, dass die Wirkung der Druck- oder Stoßwellen im menschlichen Körper, insbesondere die therapeutische Wirkung auf Gewebe, weiter verbessert wird.

Erfindungsgemäß wird diese Aufgabe hinsichtlich des eingangs genannten Geräts durch die kennzeichnenden Merkmale des Patentanspruchs 1 gelöst.

Das erfindungsgemäße Gerät unterscheidet sich von dem bekannten Gerät somit dadurch, dass in dem zu behandelnden Körperareal nicht nur die mechanische Wirkung der Druck- oder Stoßwellen entfaltet wird, sondern zumindest eine zusätzliche Wirkung, die auf Strom, Licht und/oder Ultraschall beruht. Auch kann ein weiterer mechanischer Wirkungsparameter über die Druck- und Stoßwellen hinaus erzeugt und in den Körper eingekoppelt werden, wie später noch beschrieben wird. Der Erfindung liegt die Erkenntnis zugrunde, dass die Wirkung der mechanischen Druck- oder Stoßwellen verbessert werden kann, wenn auf das zu behandelnde Körperareal, beispielsweise Weichteilgewebe, mittels Strom, Licht und/oder Ultraschall eingewirkt wird, was die therapeutische mechanische Druck- oder Stoßwellenwirkung bspw. durch eine stärkere Durchblutung und Zellstimulation des zu behandelnden Gewebes erhöht.

In bevorzugten Ausgestaltungen wird der zumindest eine weitere Wirkungsparameter durch das Applikatorteil selbst, durch eine distalseitige Kappe des Applikatorteils und/oder ein gehäusefestes Applikatorhalteteil erzeugt und von diesem bzw. dieser in den Körper eingekoppelt.

Dazu ist in praktischen Ausgestaltungen vorzugsweise vorgesehen, dass das Applikatorteil zumindest eine mit Strom beaufschlagbare Elektrode aufweist, wobei dies auch den Fall umfasst, dass das Applikatorteil selbst als Elektrode dient, dass das Appliaktorteil distalseitig eine Kappe aufweist, die zumindest eine mit Strom beaufschlagbare Elektrode aufweist, und/oder dass die zumindest eine Elektrode in oder an einem Gehäusekopfstück angeordnet ist.

Die Elektrode ist vorzugsweise kreisförmig ausgebildet, um eine großflächige Einkopplung des Stroms, der beispielsweise ein therapeutischer Wechselstrom ist, und damit eine großflächige Einwirkung auf das zu behandelnde Körperareal zu erreichen. Der Stromrückfluss kann kapazitiv ohne Gegenelektrode, über eine Neutralelektrode am Körper des Patienten oder über eine Gegenelektrode am Gerät, beispielsweise am Applikatorteil oder am Gehäuse, erfolgen.

In einer weiteren bevorzugten Ausgestaltung ist das Gerät mit einer Spannungsversorgungsquelle ausgerüstet oder mit einer solchen verbunden, die die zumindest eine Elektrode mit Hochfrequenzstrom beaufschlagt.

Diese Ausgestaltung ist insbesondere bei der Behandlung von Cellulite oder Hautfalten mit dem erfindungsgemäßen Gerät vorteilhaft, da mit dem Hochfrequenzstrom Collagenfasern so weit erwärmt werden können, bis diese schrumpfen. Als Hochfrequenzstrom wird vorzugsweise bipolarer Hochfrequenzstrom verwendet, wenn die Haut zu diesem Zweck oberflächlich erwärmt werden soll, bis eine Schrumpfung der Collagenfasern erfolgt, und/oder es wird monopolarer Hochfrequenzstrom verwendet, der eine größere Tiefenwirkung erzielt und somit das Gewebe in der Tiefe so weit erwärmt, bis dort eine Schrumpfung von Collagenfasern erfolgt. Die Kombination der Behandlung des Gewebes mit Druck- oder Stoßwellen mit der vorstehend genannten Stromstimulation, die erfindungsgemäß über das Applikatorteil selbst oder durch eine distalseitige Kappe des Applikatorteils und/oder durch ein gehäusefestes Applikatorhalteteil erzeugt und von diesem bzw. dieser in den Körper eingekoppelt wird, verstärkt die positive Wirkung der Druck- und/oder Stoßwellen zur Beseitigung von Cellulite oder Falten. Ebenso kann diese Ausgestaltung für den Fettabbau (Lipolyse) eingesetzt werden.

Der Hochfrequenzstrom weist vorzugsweise eine Frequenz im Bereich von etwa 0,5 MHz bis etwa 50 MHz auf. Eine hohe Frequenz von etwa 10 bis etwa 50 MHz hat den Vorteil, dass bei der Applikation von monopolarem Hochfrequenzstrom auf eine Neutralelektrode, die ansonsten am Körper angelegt werden muss, verzichtet werden kann.

Um Verbrennungen an der Hautoberfläche zu vermeiden, kann im Fall der Verwendung von Hochfrequenzstrom die Elektrode gekühlt sein, oder bei der Applikation wird die Elektrode permanent bewegt.

In weiteren bevorzugten Ausgestaltungen weist das Applikatorteil zumindest einen Ultraschallemitter auf, und/oder das Applikatorteil weist distalseitig eine Kappe auf, die zumindest einen Ultraschallemitter aufweist, und/oder der zumindest eine Ultraschallemitter ist in oder an einem Gehäusekopfstück angeordnet.

Dabei definiert/definieren der zumindest eine oder die mehreren Ultraschallemitter vorzugsweise eine kreisförmige Emitterfläche.

Der zumindest eine vorstehend genannte Ultraschallemitter erzeugt somit extrakorporal Ultraschall, der zusätzlich zu den mechanischen Druck- oder Stoßwellen, die eher niederfrequent sind, in den Körper eingekoppelt wird, so dass sich die niederfrequenten Druck- oder Stoßwellen mit den hochfrequenten Ultraschallwellen im Behandlungsareal überlagern können.

In weiteren bevorzugten Ausgestaltungen weist das Applikatorteil zumindest eine Lichtquelle auf, und/oder das Applikatorteil weist distalseitig eine Kappe auf, die zumindest eine Lichtquelle aufweist, und/oder in oder an einem Gehäusekopfstück ist zumindest eine Lichtquelle angeordnet, die aus der Gruppe ausgewählt ist, die eine LED, einen Laser oder eine Laser-LED enthält.

Dabei ist es wiederum bevorzugt, wenn die eine oder mehreren Lichtquellen eine kreisförmige Lichtabstrahlfläche definiert/definieren.

Auch mittels der Überlagerung von Licht mit den mechanischen Druck- oder Stoßwellen im zu behandelnden Körperareal lassen sich bessere Therapieerfolge erreichen als mit mechanischen Druck- oder Stoßwellen allein. Die Wirkung des von der zumindest einen Lichtquelle emittierten Lichts besteht ebenfalls in einer stärkeren Durchblutung und Zellstimulation des Gewebes des Behandlungsareals, wodurch die mechanischen Druck- oder Stoßwellen, die gleichzeitig eingekoppelt werden, eine bessere Wirkung entfalten. Es versteht sich, dass die Lichtquelle eine Mehrzahl von LEDs oder Laser-LEDs aufweisen kann, die beispielsweise ringförmig im oder am Applikator oder an der Kappe des Applikators angeordnet sein können.

Bei der kombinierten Applikation von mechanischen Druck- oder Stoßwellen und Licht ist es wiederum im Falle der Verwendung des Gerätes zur Behandlung von Cellulite oder Falten vorteilhaft, wenn das von der Lichtquelle applizierte Licht in der Lage ist, die Haut oberflächlich oder in der Tiefe so weit zu erwärmen, bis eine Schrumpfung von Collagenfasern erfolgt. Auch kann diese kombinierte Applikation zum Fettabbau (Lipolyse) eingesetzt werden.

Eine solche Erwärmung kann mittels des applizierten Lichtes vorteilhafterweise dadurch erreicht werden, dass die zumindest eine Lichtquelle Infrarotlicht abstrahlt. Als Lichtquelle können für diesen Zweck eine oder mehrere Infrarot-LED's oder Infrarot-Laser-LED's verwendet werden. Das Infrarotlicht ist im Unterschied zu kurzwelligerem Licht in der Lage, wie oben im Zusammenhang mit der Applikation von Hochfrequenzstrom beschrieben, eine solche Erwärmung der Haut bzw. des darunter liegenden Gewebes zu erreichen, dass Collagenfasern schrumpfen. Die hierfür erforderlichen Temperaturen liegen in einem geschätzten Bereich von etwa 50° bis etwa 80°C.

Insbesondere die zuvor genannten Ultraschallemitter oder zuvor genannten Lichtquellen sind vorzugsweise im oder am Gehäusekopfstück des Geräts in der Nähe des Applikatorteils angeordnet, was insbesondere bei auswechselbaren Applikatorteilen einen geringeren Kostenaufwand bei der Wartung des Gerätes bedeutet. Das Gehäusekopfstück ist vorzugsweise abnehmbar und dient vorzugsweise als Applikatorhalteteil zum Festhalten desselben am Handstück.

Gemäß einem weiteren Aspekt der Erfindung, der auch ohne die Merkmale des Kennzeichens des Anspruchs 1 als eigenständige Erfindung angesehen wird, werden die Stöße, mit denen die proximale Seite des Applikatorteils wiederholt beaufschlagbar ist, durch zumindest ein Schlagteil bewirkt, das entlang einer Beschleunigungsstrecke bewegt wird.

In einer vorteilhaften Ausgestaltung dieses Aspekts ist das Schlagteil aus einem Material geringen spezifischen Gewichts, beispielsweise aus Kunststoff oder einem Leichtmetall, beispielsweise aus Titan, Aluminium oder dergleichen gefertigt.

Vorzugweise weist das Schlagteil eine Masse von weniger als 10 g, vorzugsweise von weniger als 5 g, weiter vorzugsweise von weniger als 3 g auf.

Die Ausgestaltung des zumindest einen Schlagteils mit geringem Gewicht hat den Vorteil, dass im Unterschied zu dem bekannten Gerät Pulsfrequenzen bzw. Stoßfrequenzen von mehr als 20 Hz erreicht werden können, ohne dass es dazu erforderlich ist, beispielsweise die Beschleunigungsstrecke des Schlagteils zu vergrößern. Dadurch bleibt das Gerät handlich, und es ist nicht erforderlich, im Falle eines pneumatischen Antriebs des Schlagteils den Druck des Pneumatikantriebes zu erhöhen. Eine höhere Puls- oder Stoßfrequenz hat den Vorteil, dass Resonanzen im zu behandelnden Gewebe effizienter angeregt werden können, wobei derartige Resonanzen die therapeutische Wirkung der Druck- oder Stoßwellen weiter verbessern.

Weiterhin ist es bevorzugt, wenn das Schlagteil Ausnehmungen, Aushöhlungen, Vertiefungen oder Öffnungen aufweist.

Die Ausnehmungen, Aushöhlungen, Vertiefungen oder Öffnungen führen ebenfalls zu einer Gewichtsreduktion des Schlagteils, ohne dass ein besonders leichtes Material wie die vorstehend genannten für die Fertigung des Schlagteils verwendet werden müssen. So kann im Rahmen der vorliegenden Ausgestaltung das Schlagteil auch aus einem Metall höherer Dichte und höherer Härte und somit verschleißfester, bspw. aus Stahl, gefertigt werden, wobei die Ausnehmungen, Aushöhlungen, Vertiefungen oder Öffnungen dann die Masse des Schlagteils deutlich reduzieren.

Weiterhin ist es bevorzugt, wenn das Schlagteil auf eine Aufprallgeschwindigkeit beim Aufprall auf das Applikatorteil beschleunigt wird, die größer als 20 m/s ist.

Eine höhere Aufprallgeschwindigkeit des Schlagteils von mehr als 20 m/s, die bei dem bekannten Gerät nicht erreicht wird, hat den Vorteil, dass zum einen wiederum die Pulsfrequenz der Stoßerzeugung erhöht werden kann, und zum anderen wird mit der höheren Aufprallgeschwindigkeit trotz der geringeren Masse des Schlagteils gemäß den zuvor genannten Ausgestaltungen eine ausreichend hohe Druckwellenenergie im Applikatorteil und somit nach der Einkopplung im zu behandelnden Körperareal erreicht.

In einer weiteren bevorzugten Ausgestaltung weist das Schlagteil eine Länge auf, die im Wesentlichen nicht länger als der zweifache Durchmesser des Schlagteils ist.

Eine möglichst kurze Ausgestaltung des Schlagteils hat wiederum den Vorteil einer geringeren Masse des Schlagteils zur Erzielung einer höheren Pulsfrequenz, wobei eine Länge, die etwa dem zweifachen Durchmesser des Schlagteils entspricht, vorteilhafterweise eine Verkippung bzw. Verkantung des Schlagteils auf seiner Beschleunigungsstrecke vermeidet.

Gemäß einem noch weiteren Aspekt der Erfindung, der auch ohne die Merkmale des Kennzeichen des Anspruchs 1 oder die vorstehend genannten Merkmale als eigenständige Erfindung angesehen wird, werden die Stöße, mit denen die proximale Seite des Applikatorteils wiederholt beaufschlagbar ist, durch zumindest zwei Schlagteile bewirkt, die entlang jeweils einer Beschleunigungsstrecke bewegt werden.

Der Vorteil eines Druck- oder Stoßwellenerzeugungsmechanismus mit einer Mehrzahl von Schlagteilen besteht darin, dass die einzelnen Schlagteile für sich genommen jeweils mit geringer Masse ausgeführt werden können, ohne den Gesamtimpuls auf das Applikatorteil zu reduzieren. Die mehreren Schlagteile bewirken somit eine Pulsverstärkung bei gleichzeitig geringer Schlagteilmasse und nicht verlängerter Beschleunigungsstrecke.

Die Schlagteile können dabei im Wesentlichen gleichzeitig auf das Applikatorteil aufprallen, um einen möglichst hohen Gesamtimpuls auf das Applikatorteil auszuüben, wodurch Druck- oder Stoßwellen mit hoher Energie erzeugt werden können, oder die Schlagteile können auch nacheinander auf das Applikatorteil aufprallen, wodurch der Vorteil einer höheren Pulsfrequenz erreicht werden kann.

Im letzteren Fall ist es weiterhin bevorzugt, wenn das Applikatorteil aus seiner Längsachse verkippbar ist, und die Schlagteile außerhalb der Längsachse auf das Applikatorteil aufprallen.

Bei dieser Ausgestaltung bewirken die nacheinander und außerhalb der Längsachse auf das aus seiner Längsachse verkippbare Applikatorteil aufprallenden Schlagteile eine Kipp- oder Taumelbewegung des Applikatorteils, je nachdem, ob zwei Schlagteile oder drei oder mehr Schlagteile vorhanden sind. Die Kipp- oder Taumelbewegung des Applikatorteils bewirkt zusätzlich zur Einkopplung der Druck- oder Stoßwellen eine Massagewirkung auf das Gewebe. Diese Ausgestaltung stellt insbesondere eine vorteilhafte Ausgestaltung des oben genannten Aspekts der Erfindung dar, wonach das Gerät zusätzlich zur extrakorporalen Erzeugung zumindest eines weiteren Wirkungsparameters und Einkopplung desselben in dem Körper ausgebildet ist, wobei der Wirkungsparameter eine mechanische Wirkung umfasst.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: ein medizinisches Gerät zur Behandlung des menschlichen oder tierischen Körpers mit mechanischen Druck- oder Stoßwellen in einer schematischen Längsschnittdarstellung;
- Fig. 2: eine Ansicht auf das distale Ende eines Applikatorteils des Geräts in Fig. 1 gemäß einem Ausführungsbeispiel;
- Fig. 3: eine Ansicht auf das distale Ende eines Applikatorteils des Geräts in Fig. 1 gemäß einem weiteren Ausführungsbeispiel;
- Fig. 4: eine Ansicht auf das distale Ende eines Applikatorteils des Geräts in Fig. 1 gemäß einem noch weiteren Ausführungsbeispiel;
- Fig. 5: ein weiteres Ausführungsbeispiel eines medizinischen Geräts zur Behandlung des menschlichen oder tierischen Körpers mit mechanischen Druckoder Stoßwellen in einer schematischen Längsschnittdarstellung; und
- Fig. 6: ein noch weiteres Ausführungsbeispiel eines medizinischen Geräts zur Behandlung des menschlichen oder tierischen Körpers mit mechanischen Druck- oder Stoßwellen in einer schematischen Längsschnittdarstellung.

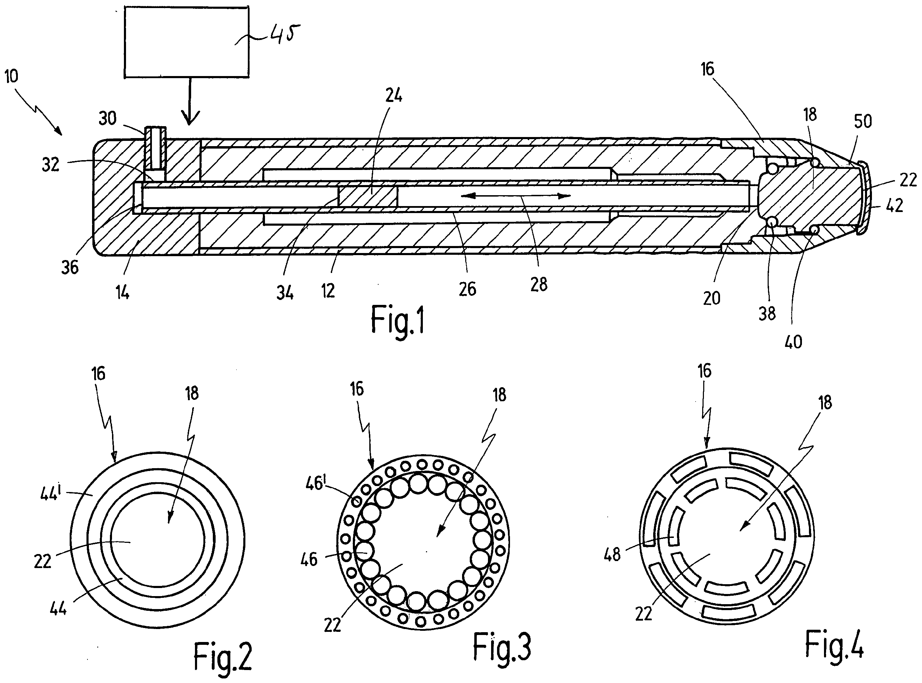

In Fig. 1 ist ein mit dem allgemeinen Bezugszeichen 10 versehenes medizinisches Gerät zur Behandlung des menschlichen oder tierischen Körpers mit mechanischen Druck- oder Stoßwellen dargestellt. Das Gerät 10 wird beispielsweise für die Schmerztherapie oder allgemein zur Weichgewebebehandlung verwendet.

Das Gerät 10 weist allgemein ein Gehäuse 12 mit einem proximalen Endstück 14 und einem distalen Kopfstück 16 auf. Das Gerät 10 ist in Form eines stabförmigen Handstücks ausgebildet.

Das Gerät 10 weist eine Vorrichtung zur Erzeugung von Druck- oder Stoßwellen auf, die ein am distalen Ende des Gehäuses 12 im distalen Kopfstück 16 angeordnetes Applikatorteil 18 aufweist. Das Kopfstück 16 dient als Applikatorhalter zum Festhalten des Applikatorteils 18 am Handstück.

Das Applikatorteil 18 weist eine proximale Eintrittsfläche 20 und eine distale Austrittsfläche 22 für die Druck- oder Stoßwellen auf. Über die Eintrittsfläche 20 werden in dem Applikatorteil 18 die Druck- oder Stoßwellen erzeugt. Dazu ist in dem Gehäuse 12 ein Schlagteil 24 angeordnet, das in einem in Längsrichtung des Gehäuses 12 verlaufenden Führungsrohr 26 gemäß einem Doppelpfeil 28 hin und her bewegbar ist, um auf die Eintrittsfläche 20 des Applikatorteils 18 wiederholt einen Stoß auszuüben. Das Schlagteil 24 wird mittels Druckluft nach distal bewegt, wozu am proximalen Endstück 14 des Gehäuses 12 ein Drucklufteinlass 30 angeordnet ist, durch den Druckluft einer nicht dargestellten externen Druckluftquelle in das Führungsrohr 26 durch eine Öffnung 32 desselben impulsartig eingeleitet wird, wobei die eingeleitete Druckluft auf eine proximale Fläche 34 des Schlagteils 24 wirkt und dadurch das Schlagteil 24 aus einer Ausgangsstellung bei 36 zu dem Applikatorteil 18 hin beschleunigt wird.

Bei jedem Auftreffen des Schlagteils 24 auf die Eintrittsfläche 20 des Applikatorteils 18 wird in diesem eine Druck- oder Stoßwelle induziert, die sich durch das Applikatorteil 18 zur Austrittsfläche 22 hindurch ausbreitet und von dieser austritt, um in den Körper eines Patienten eingekoppelt zu werden.

Das Applikatorteil 18 führt bei jedem Stoß des Schlagteils 24 nur einen minimalen Hub aus, wobei das Schlagteil 18 zwischen einem proximalen elastomeren O-Ring 38 und einem distalen elastomeren O-Ring 40 elastisch eingespannt ist. Das Applikatorteil 18 kann andererseits jedoch auch so gelagert sein, dass es einen vergleichsweise großen Hub von bis zu 5 mm ausführen kann.

Das Gerät 10 ist nicht nur zur Erzeugung und Einkopplung mechanischer Druck- oder Stoßwellen ausgelegt, sondern es ist zusätzlich zur extrakorporalen Erzeugung zumindest eines weiteren physiologischen Wirkungsparameters und Einkopplung desselben in den menschlichen oder tierischen Körper ausgebildet, wobei der Wirkungsparameter einen elektrischen Strom, elektromagnetische Wellen, insbesondere Licht, Ultraschall, und/oder eine weitere mechanische Wirkung enthält.

Vorzugsweise wird der zumindest eine weitere Wirkungsparameter vom Applikatorteil 18 oder von einer an der distalen Austrittsfläche 22 angeordneten Kappe 42 und/oder von dem Gehäusekopfstück 16, insbesondere in einen distalen Bereich desselben, erzeugt und in den Körper eines Patienten eingekoppelt.

Fig. 2 zeigt eine Ausgestaltung des Applikatorteils 18, bei der das Applikatorteil 18 an seiner Austrittsfläche 22 eine mit Strom beaufschlagbare Elektrode 44 aufweist. Alternativ hierzu kann jedoch auch die zuvor erwähnte Kappe 42 die Elektrode 44 aufweisen.

Die Elektrode 44 ist insbesondere kreisringförmig oder scheibenförmig ausgebildet und weist in dem gezeigten Ausführungsbeispiel die Form eines Kreisrings auf. Es kann auch das gesamte Applikatorteil 18 oder ein Teil desselben selbst als Elektrode ausgebildet sein. Wie in Fig. 2 ebenfalls dargestellt ist, kann zusätzlich oder alternativ das Gehäusekopfstück 16 eine Elektrode 44' aufweisen oder selbst als solche ausgebildet sein.

Über die Elektrode 44 und/oder 44' kann zusätzlich zur Einkopplung der mechanischen Druck- oder Stoßwellen ein Strom, insbesondere ein therapeutischer Wechselstrom in den Körper in das Behandlungsareal eingekoppelt werden, so dass sich die Wirkung der mechanischen Druck- oder Stoßwellen und des Stroms im Behandlungsareal überlagern.

Über die Elektrode 44 und/oder 44' kann jedoch auch anstelle von therapeutischem Wechselstrom ein monopolarer oder bipolarer Hochfrequenzstrom in den Körper in das Behandlungsareal eingekoppelt werden, der in der Lage ist, die Haut oberflächlich oder in der Tiefe so weit zu erwärmen, dass Collagenfasern schrumpfen. Dieser Effekt ist bei der Behandlung von Cellulite oder Falten besonders vorteilhaft. Eine Spannungsversorgungsquelle 45, die entsprechend bipolaren oder monopolaren Hochfrequenzstrom bereitstellt, ist in Fig. 1 schematisch dargestellt. Die Hochfrequenzspannung wird entsprechend an die Elektrode 44 und/oder 44' angelegt. Im Fall der Applikation von bipolarem Hochfrequenzstrom kann die eine der beiden Elektroden 44, 44' die Neutralelektrode und die andere der beiden Elektroden die Aktivelektrode bilden. Der Stromfluss findet dann zwischen diesen beiden Elektroden mit geringer Eindringtiefe in die Haut statt.

Im Fall der Applikation von monopolarem Hochfrequenzstrom können eine oder beide Elektroden 44, 44' jeweils die Aktivelektrode bilden, während eine nicht dargestellte Neutralelektrode am Körper des Patienten, üblicherweise fernab von der Behandlungsstelle, appliziert wird. Auf eine solche zusätzliche Neutralelektrode kann jedoch verzichtet werden, wenn der Hochfrequenzstrom mit einer hohen Frequenz appliziert wird, bspw. im Bereich von etwa 10 MHz bis etwa 50 MHz. Die Frequenz des Hochfrequenzstroms kann jedoch allgemein auch niedriger sein, bspw. im Bereich von etwa 0,5 bis 4 MHz.

Fig. 3 zeigt ein Ausführungsbeispiel des Applikatorteils 18, bei dem das Applikatorteil 18 eine Mehrzahl an Lichtquellen 46 aufweist, die zusätzlich zu den mechanischen Druck- oder Stoßwellen Licht, insbesondere therapeutisches Licht, in das Behandlungsareal des Körpers des Patienten einkoppeln. Alternativ oder zusätzlich können Lichtquellen 46' am Gehäusekopfstück im oder am distalen Bereich 50 angeordnet sein.

Die Lichtquellen 46 oder 46' bestehen in dem gezeigten Ausführungsbeispiel aus einem Array von Leuchtdioden (LED's), können jedoch auch einen Laser oder Laser-LED's umfassen.

Wenn das Gerät 10 zur Behandlung von Cellulite oder Falten eingesetzt werden soll, sind die Lichtquellen 46 oder 46' vorzugsweise solche, die Infrarotlicht abstrahlen. Das Infrarotlicht führt ebenso wie der oben genannte Hochfrequenzstrom zu einer Gewebserwärmung, die die Wirkung hat, dass Haut- bzw. Collagenfasern zum Schrumpfen gebracht werden, d.h. zumindest teilweise eine Denaturierung dieser Fasern zu bewirken. In diesem Fall können als Lichtquellen 46 oder 46' Infrarot-LED's oder Infrarot-Laser-LED's verwendet werden.

Die Lichtquellen 46 oder 46' sind kreisförmig angeordnet. Ebenso ist es wiederum möglich, die Lichtquelle oder die Lichtquellen 46 an der Kappe 42 vorzusehen.

Fig. 4 zeigt ein Ausführungsbeispiel des Applikatorteils 18, bei der auf der Austrittsfläche 22 eine Mehrzahl an Ultraschallemittern 48 angeordnet sind. Die Ultraschallemitter 48 erzeugen Ultraschall, der dann von den Ultraschallemittern 48 bzw. dem Applikatorteil 18 zusätzlich zu den mechanischen Druck- oder Stoßwellen in den Körper des Patienten eingekoppelt werden.

Die Ultraschallemitter 48 sind in dem gezeigten Ausführungsbeispiel kreisförmig an der Austrittsfläche 22 des Applikatorteils 18 angeordnet, bzw. definieren eine kreisförmige Ultraschallabstrahlfläche. Die Ultraschallemitter 48 können ebenso wiederum an der Kappe 42 angeordnet sein, oder das Gehäusekopfstück 16 kann Ultraschallemitter 48' im oder am distalen Bereich 50 aufweisen.

Es versteht sich, dass die Ausführungsbeispiele gemäß Fig. 2 bis 4 auch miteinander kombiniert werden können, d.h. das Applikatorteil 18, die Applikatorkappe 42 und/oder das Gehäusekopfstück 16 können Kombinationen aus der Elektrode 44, den Lichtquellen 46 und/oder den Ultraschallemittern 48 aufweisen.

In den Fällen, bei denen die Elektrode(n), Lichtquelle(n), Ultraschallemitter im oder am Applikatorteil 18 vorgesehen sind, kann die Energieversorgung dieser Elemente über Schleifkontakte, induktiv oder kapazitiv erfolgen.

Wieder mit Bezug auf Fig. 1 ist das Schlagteil 24 aus einem Material geringen spezifischen Gewichts gefertigt. Ein solches Material kann insbesondere Kunststoff oder ein Leichtmetall, beispielsweise Titan, Aluminium oder dergleichen, sein. Durch die Verwendung eines Materials geringen spezifischen Gewichts weist das Schlagteil 24 eine geringe Masse auf, so dass das Schlagteil 24 ohne Vergrößerung der Beschleunigungsstrecke in dem Führungsrohr 26 mit einer hohen Geschwindigkeit von mehr als 20 m/s auf die Eintrittsfläche 20 des Applikatorteils 18 aufprallt.

Die Masse des Schlagteils 24 beträgt vorzugsweise weniger als 10 g, vorzugsweise weniger als 5 g, weiter vorzugsweise weniger als 3 g.

Die Masse des Schlagteils 24 kann weiter verringert werden, indem in dem Schlagteil 24 Ausnehmungen, Aushöhlungen, Vertiefungen oder Öffnungen vorgesehen sind, wobei das Schlagteil 24 beispielsweise im einfachsten Fall eine durchgehende oder blinde längs verlaufende Bohrung aufweisen kann, so dass das Schlagteil 24 insgesamt oder teilweise hülsenförmig ausgebildet ist. Die Öffnungen können auch in Richtung quer zur Längsrichtung des Schlagteils 24 in diesem ausgebildet sein.

Damit das Schlagteil 24 in dem Führungsrohr 26 nicht verkantet, ist seine Länge in Richtung des Doppelpfeils 28 größer oder gleich seinem zweifachen Durchmesser in Richtung quer zu dem Doppelpfeil 28, wobei die Länge des Schlagteils 24 vorzugsweise etwa gleich seinem doppelten Durchmesser ist.

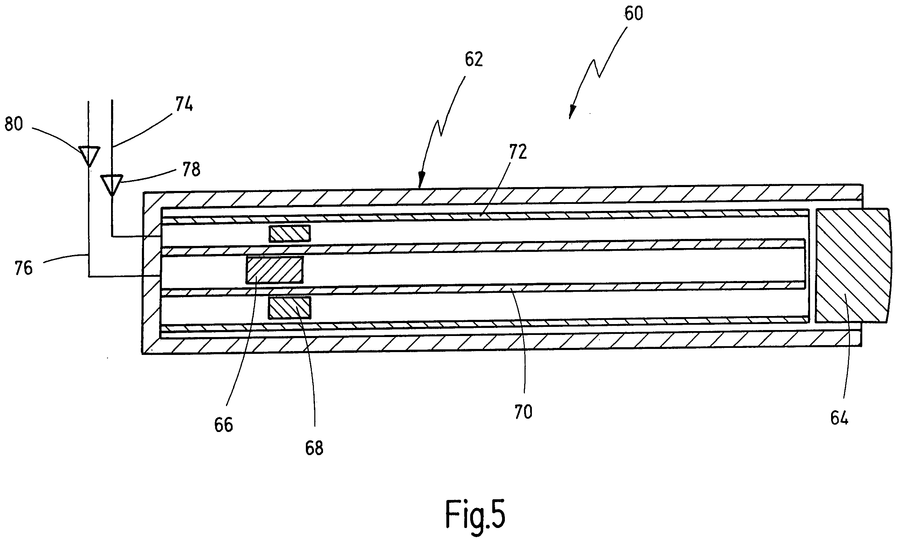

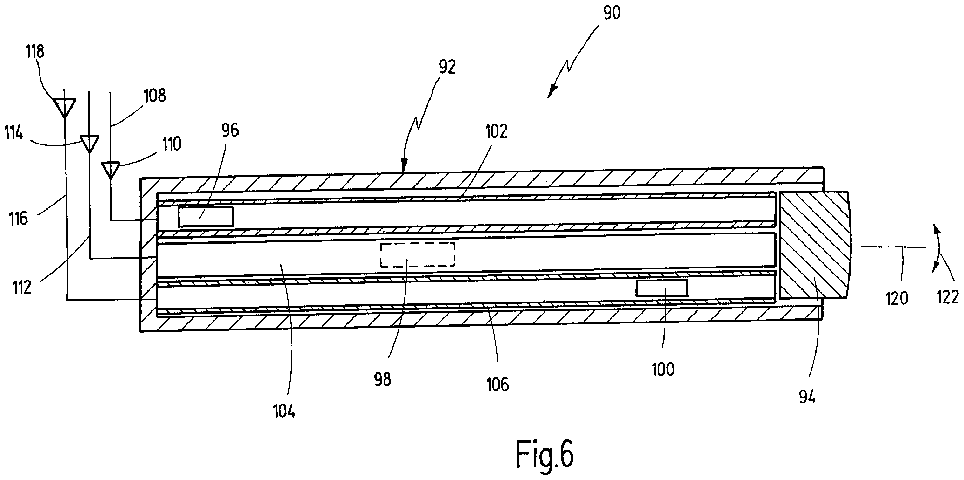

Mit Bezug auf Fig. 5 und 6 werden weitere Ausführungsbeispiele eines medizinischen Geräts zur Behandlung des menschlichen oder tierischen Körpers mit mechanischen Druck- oder Stoßwellen beschrieben.

Fig. 5 zeigt ein medizinisches Gerät 60 mit einem Gehäuse 62 und einem Applikatorteil 64 zur Erzeugung und Einkopplung von Druck- oder Stoßwellen in den menschlichen oder tierischen Körper. Das Applikatorteil 64 kann eine oder mehrere der Ausgestaltungen gemäß Fig. 2 bis 4 aufweisen, wozu auf die obige Beschreibung verwiesen wird. Im Unterschied zu dem Gerät 10 weist das Gerät 60 eine Vorrichtung zur Erzeugung von Druck- oder Stoßwellen auf, die neben dem Applikatorteil 64 zwei Schlagteile 66 und 68 aufweist. Das Schlagteil 66 entspricht dem Schlagteil 24 in Fig. 1, wobei das Schlagteil 66 in einem Führungsrohr 70 hin und her bewegt wird, das dem Führungsrohr 26 in Fig. 1 entspricht.

Das Schlagteil 68 ist in dem gezeigten Ausführungsbeispiel in Fig. 5 als Ringhülse ausgebildet, die konzentrisch um das Schlagteil 66 herum angeordnet ist, und in einem Führungsrohr 72 hin und her bewegt wird, das das Führungsrohr 70 konzentrisch umgibt und als zylindrisches Rohr ausgebildet ist.

Bei dem Ausführungsbeispiel in Fig. 5 werden das Schlagteil 66 und 68 über Druckluftleitungen 74 und 76 mit entsprechenden Steuerventilen 78 und 80 so mit Druck beaufschlagt, dass die Schlagteile 66 und 68 gleichzeitig auf das Applikatorteil 64 aufprallen. Damit die Schlagteile 66 und 68 synchron beschleunigt werden, sind ihre Massen aufeinander abgestimmt oder der Druck der Luft oder des Gases, mit der bzw. dem die Schlagteile 66 und 68 proximal impulsartig beaufschlagt werden, ist entsprechend auf die beiden Schlagteile 66 und 68 abgestimmt.

Durch das Vorhandensein zumindest zweier Schlagteile 66 und 68, die gleichzeitig auf das Applikatorteil 64 aufprallen, wird eine entsprechend höhere Energie der Druck- oder Stoßwellen in dem Applikatorteil 64 erzeugt und in den Körper des Patienten eingekoppelt.

In Fig. 6 ist ein medizinisches Gerät 90 zur Behandlung des menschlichen oder tierischen Körpers mit mechanischen Druck- oder Stoßwellen dargestellt, das ein Gehäuse 92 und am distalen Ende ein Applikatorteil 94 aufweist.

Das Gerät 90 weist eine Vorrichtung zur Erzeugung von Druck- oder Stoßwellen auf, die drei oder vier Schlagteile 96, 98 und 100 aufweist.

Das Schlagteil 96 ist in einem Führungsrohr 102, das Schlagteil 98 in einem Führungsrohr 104 und das Schlagteil 100 in einem Führungsrohr 106 hin und her bewegbar. Das Führungsrohr 102 ist über eine Druckzuführleitung 108 mit Steuerventil 110, das Führungsrohr 104 über eine Druckzuführleitung 112 mit Steuerventil 114 und das Führungsrohr 106 über eine Druckzuführleitung 116 mit Steuerventil 118 mit Druck beaufschlagbar, um das jeweilige Schlagteil 96, 98 und 100 von einer proximalen Ausgangsstellung zum Applikatorteil 94 hin zu beschleunigen.

Im Unterschied zu dem Ausführungsbeispiel in Fig. 5 treffen die Schlagteile 96, 98 und 100 bei dem Gerät 90 nacheinander auf das Applikatorteil 94. Hierdurch kann eine höhere Schlag- bzw. Pulsfrequenz der Erzeugung der mechanischen Druck- oder Stoßwellen erreicht werden, insbesondere eine Schlag- oder Impulsfrequenz von mehr als 30 Hz oder gar mehr als 100 Hz.

Bei diesem Ausführungsbeispiel kann es weiter vorgesehen sein, dass das Applikatorteil 94 in dem Gehäuse 92 bezüglich einer Längsmittelachse 120 des Applikatorteils 94 verkippbar gelagert ist, wie mit einem Doppelpfeil 122 veranschaulicht ist. In diesem Fall sind die Schlagteile 96, 98, 100 bzw. die Führungsrohre 102, 104, 106 bezüglich der Längsmittelachse 120 außermittig angeordnet, so dass die Schlagteile 96, 98, 100 außerhalb der Längsmittelachse 120 auf das Applikatorteil 94 aufprallen und ein entsprechendes Verkippen des Applikatorteils 94 bezüglich der Längsmittelachse 120 bewirken. Durch das periodische Hintereinander erfolgende Aufprallen der Schlagteile 96, 98 und 100 auf das Applikatorteil 94 führt dieses eine Art Taumelbewegung um die Längsmittelachse 120 aus. Diese Taumelbewegung stellt eine weitere mechanische Wirkung dar, die zusätzlich zu den mechanischen Druck- oder Stoßwellen, die ebenfalls von den Schlagteilen 96, 98 und 100 in dem Applikatorteil 94 erzeugt werden, in den Körper des Patienten eingekoppelt werden können, wodurch eine zusätzliche Massagewirkung durch das Applikatorteil 94 erzeugt wird.

In dem gezeigten Ausführungsbeispiel gemäß Fig. 6 können die Applikatorteile 96, 98, 100 bzw. die Führungsrohre 102, 104, 106 um die Längsmittelachse 120 in einem Winkelabstand von 120° zueinander angeordnet sein. Es können auch vier oder mehr Schlagteile vorgesehen sein, die entsprechend in einer Winkelverteilung von 90° oder einer entsprechenden Winkelverteilung um die Längsmittelachse 120 herum angeordnet sind, so dass sie jeweils außermittig auf das Schlagteil 94 aufprallen.

Die Steuerventile 110, 114, 118 in den Druckzufuhrleitungen 108, 112, 116 werden entsprechend getaktet, um die Schlagteile 96, 98, 100 einzeln und nacheinander auf das Applikatorteil 94 aufprallen zu lassen.

Des weiteren kann das Applikatorteil 94 zusätzlich gemäß einer oder mehreren der Ausgestaltungen in Fig. 2 bis 4 ausgestaltet sein.

## Patentansprüche

1. Medizinisches Gerät zur Behandlung des menschlichen oder tierischen Körpers mit mechanischen Druck- oder Stoßwellen, mit einer Vorrichtung zur Erzeugung der Druck- oder Stoßwellen, die zumindest ein Applikatorteil (18; 64; 94) aufweist, das zur Erzeugung der Druck- oder Stoßwellen auf einer proximalen Seite mit Stößen beaufschlagbar ist, und das auf einer distalen Seite die Druck- oder Stoßwellen in den Körper einkoppelt, **dadurch gekennzeichnet, dass** das Gerät (10; 60; 90) zusätzlich zur extrakorporalen Erzeugung zumindest eines weiteren physiologischen Wirkungsparameters zur Behandlung des menschlichen oder tierischen Körpers und kombinierten Einkopplung desselben mit den Druck- oder Stoßwellen in den Körper ausgebildet ist, der aus der Gruppe ausgewählt ist, die elektrischen Strom, Licht und/oder Ultraschall enthält, wobei das Gerät aufweist:
- zumindest eine mit Strom beaufschlagbare Elektrode (44, 44') und eine Spannungsversorgungsquelle, die die zumindest eine Elektrode (44, 44') mit einem monopolaren oder bipolaren Hochfrequenzstrom beaufschlagt, wobei der Hochfrequenzstrom eine Frequenz im Bereich von etwa 0,5 MHz bis etwa 50 MHz aufweist, und/oder
- zumindest einen Ultraschallemitter (48, 48'), wobei der Ultraschallemitter (48, 48') Ultraschallwellen emittiert, die hochfrequent sind, während die Druck- oder Stoßwellen niederfrequent sind, und/oder
- zumindest eine Lichtquelle (46, 46'), die aus der Gruppe ausgewählt ist, die eine LED, einen Laser, eine Laser-LED enthält, wobei die zumindest eine Lichtquelle (46, 46') eine Infrarotlicht abstrahlende Lichtquelle ist.

2. Gerät nach Anspruch 1, **dadurch gekennzeichnet, dass** das zumindest eine Applikatorteil (18) selbst als die mit Strom beaufschlagbare Elektrode (44, 44') ausgebildet ist.

3. Gerät nach Anspruch 1 oder 2, **dadurch gekennzeichnet, dass** das Applikatorteil (18), eine distalseitig am Applikatorteil (18) angeordnete Kappe (42) oder ein Gehäusekopfstück (16) die zumindest eine Elektrode (44, 44'), den zumindest einen Ultraschallemitter (48, 48') und/oder die zumindest eine Lichtquelle (46, 46') aufweist.

4. Gerät nach einem der Ansprüche 1 bis 3, **dadurch gekennzeichnet, dass** die zumindest eine Elektrode (44, 44') kreisförmig ist, und/oder dass der zumindest eine oder die mehreren Ultraschallemitter (48, 48') eine kreisförmige Emitterfläche definiert/definieren, und/oder dass die zumindest eine oder die mehreren Lichtquellen (46, 46') eine kreisförmige Lichtabstrahlfläche definiert/definieren.

5. Gerät nach einem der Ansprüche 1 bis 4, **dadurch gekennzeichnet, dass** die Stöße, mit denen die proximale Seite des Applikatorteils (18) wiederholt beaufschlagbar ist, durch zumindest ein Schlagteil (24; 66, 68; 96, 98, 100) bewirkt werden, das entlang einer Beschleunigungsstrecke bewegt wird.

6. Gerät nach Anspruch 5, **dadurch gekennzeichnet, dass** das Schlagteil (24; 66, 68; 96, 98, 100) aus einem Material geringen spezifischen Gewichts, beispielsweise aus Kunststoff oder einem Leichtmetall, beispielsweise aus Titan, Aluminium oder dgl. gefertigt ist.

7. Gerät nach Anspruch 5 oder 6, **dadurch gekennzeichnet, dass** das Schlagteil (24; 66, 68; 96, 98, 100) Ausnehmungen, Aushöhlungen, Vertiefungen oder Öffnungen aufweist.

8. Gerät nach einem der Ansprüche 5 bis 7, **dadurch gekennzeichnet, dass** das Schlagteil (24; 66, 68; 96, 98, 100) auf eine Aufprallgeschwindigkeit beim Aufprall auf das Applikatorteil beschleunigt wird, die größer als 20 m/s ist.

9. Gerät nach einem der Ansprüche 5 bis 8, **dadurch gekennzeichnet, dass** die Stöße, mit denen die proximale Seite des Applikatorteils wiederholt beaufschlagbar ist, durch zumindest zwei Schlagteile (66, 68; 96, 98, 100) bewirkt werden, die entlang jeweils einer Beschleunigungsstrecke bewegt werden.

10. Gerät nach Anspruch 9, **dadurch gekennzeichnet, dass** die Schlagteile (66, 68) im wesentlichen gleichzeitig auf das Applikatorteil aufprallen.

11. Gerät nach Anspruch 9, **dadurch gekennzeichnet, dass** die Schlagteile (96, 98, 100) nacheinander auf das Applikatorteil aufprallen.

12. Gerät nach Anspruch 11, **dadurch gekennzeichnet, dass** das Applikatorteil (94) aus seiner Längsmittelachse (120) verkippbar ist, und dass die Schlagteile (96, 98, 100) außerhalb der Längsmittelachse (120) auf das Applikatorteil (94) aufprallen.

## Claims

1. Medical apparatus for treatment of the human or animal body by mechanical pressure waves or shock waves, comprising a device for generating the pressure waves or shock waves, which device has at least one applicator part (18; 64; 94) which can be subjected to impacts at a proximal end for generation of the pressure waves or shock waves, and which applies said pressure waves or shock waves into the body at a distal end, **characterized in that** the apparatus (10; 60; 90) is additionally configured to extra-corporally generate at least one further physiological action parameter for treatment of the human or animal body and to apply same into the body in combination with the pressure waves or shock waves, which action parameter is chosen from the group consisting of electrical current, electro-magnetic waves and/or ultrasound, wherein the apparatus comprises:
at least one electrode (44, 44') which can be supplied with current, and a power supply source which supplies the at least one electrode (44, 44') with a monopolar or bipolar high frequency current, wherein the high frequency current has a frequency in the range from about 0.5 MHz to about 50 MHz, and/or
at least one ultrasound emitter (48, 48'), wherein the ultrasound emitter (48, 48') emits ultrasound waves which are of high frequency, while the pressure waves or shock waves are of low frequency, and/or
at least one light source (46, 46') chosen from the group consisting of an LED, a laser, a laser-LED, wherein the at least one light source (46, 46') is an infrared light radiating light source.

2. Apparatus of claim 1, **characterized in that** the at least one applicator part (18) itself is configured as the electrode (44, 44') which can be supplied with current.

3. Apparatus of claim 1 or 2, **characterized in that** the applicator part (18), a cap (42) arranged on a distal side of the applicator part (18), or a housing headpiece (16) comprises the at least one electrode (44, 44'), the at least one ultrasound emitter (48, 48') and/or the at least one light source (46, 46').

4. Apparatus of any one of claims 1 to 3, **characterized in that** the at least one electrode (44, 44') is circular, and/or the one ultrasound emitter or the plurality of ultrasound emitters (48, 48') defines/define a circular emitter surface, and/or the at least one light source or the plurality of light sources (46, 46') defines/define a circular light emission surface.

5. Apparatus of any one of claims 1 to 4, **characterized in that** the impacts, the proximal end of the applicator part (18) can be repeatedly acted on, are effected by at least one percussion part (24; 66, 68; 96, 98, 100) that is moved along an acceleration path.

6. Apparatus of claim 5, **characterized in that** the percussion part (24; 66, 68; 96, 98, 100) is made from a material of low specific weight, for example from plastic or a light metal, for example titanium, aluminium or the like.

7. Apparatus of claim 5 or 6, **characterized in that** the percussion part (24; 66, 68; 96, 98, 100) has recesses, hollows, depressions or openings.

8. Apparatus of any one of claims 5 to 7, **characterized in that** the percussion part (24; 66, 68; 96, 98, 100) is accelerated to an impacting speed of greater than 20 m/s upon impacting said applicator part.

9. Apparatus of any one of claims 5 to 8, **characterized in that** the impacts, the proximal side of the applicator part is repeatedly supplied with, are effected by at least two percussion parts (66, 68; 96, 98, 100) which are moved along a respective acceleration path in each case.

10. Apparatus of claim 9, **characterized in that** the percussion parts (6, 68) impact substantialy simultaneously on said applicator part.

11. Apparatus of claim 9, **characterized in that** the percussion parts (96, 98, 100) impact in succession on said applicator part.

12. Apparatus of claim 1, **characterized in that** the applicator part (94) can be tilted from its longitudinal center axis (120), and that the percussion parts (96, 98, 100) impact on the applicator part (94) outside the longitudinal center axis (120).

## Revendications

1. Appareil médical servant à traiter le corps d'un être humain ou d'un animal avec des ondes de pression ou de choc mécaniques, comprenant un dispositif servant à produire des ondes de pression ou de choc, lequel présente au moins une partie d'applicateur (18 ; 64 ; 94), qui peut être soumise à l'action de chocs sur un côté proximal afin de produire les ondes de pression ou de choc et qui injecte dans le corps les ondes de pression ou de choc sur un côté distal, **caractérisé en ce que** l'appareil (10 ; 60 ; 90) est réalisé en supplément afin de produire de manière extracorporelle au moins un autre paramètre d'action physiologique servant à traiter le corps d'un être humain ou d'un animal et afin d'injecter de manière combinée ce dernier avec les ondes de pression ou de choc dans le corps, lequel paramètre d'action est choisi parmi le groupe contenant un courant électrique, une lumière et/ou un ultrason, dans lequel l'appareil présente :
- au moins une électrode (44, 44') pouvant être soumise à l'action d'un courant et une source d'alimentation en tension, qui soumet l'au moins une électrode (44, 44') à l'action d'un courant à fréquence élevée monopolaire ou bipolaire, dans lequel le courant à fréquence élevée présente une fréquence située dans la plage allant d'environ 0,5 MHz à environ 50 MHz, et/ou
- au moins un émetteur d'ultrasons (48, 48'), dans lequel l'émetteur d'ultrasons (48, 48') émet des ultrasons à haute fréquence, tandis que les ondes de pression ou de choc sont à basse fréquence, et/ou
- au moins une source de lumière (46, 46'), qui est choisie parmi le groupe contenant une DEL, un laser, une DEL à laser, dans lequel l'au moins une source de lumière (46, 46') est une source de lumière émettant une lumière infrarouge.

2. Appareil selon la revendication 1, **caractérisé en ce que** l'au moins une partie d'applicateur (18) est elle-même réalisée sous la forme de l'électrode (44, 44') pouvant être soumise à l'action du courant.

3. Appareil selon la revendication 1 ou 2, **caractérisé en ce que** la partie d'applicateur (18) présente un capuchon (42) disposé côté distal au niveau de la partie d'applicateur (18) ou une pièce de tête de boîtier (16), l'au moins une électrode (44, 44'), l'au moins un émetteur d'ultrasons (48, 48') et/ou l'au moins une source de lumière (46, 46').

4. Appareil selon l'une quelconque des revendications 1 à 3, **caractérisé en ce que** l'au moins une électrode (44, 44') est de forme circulaire, et/ou que l'au moins un ou les nombreux émetteurs d'ultrasons (48, 48') définit/définissent une surface d'émission de forme circulaire, et/ou que l'au moins une source de lumière ou les nombreuses sources de lumière (46, 46') définit/définissent une surface d'émission de lumière de forme circulaire.

5. Appareil selon l'une quelconque des revendications 1 à 4, **caractérisé en ce que** les chocs, à l'action desquels le côté proximal de la partie d'applicateur (18) peut être soumis de manière répétée, sont provoqués par au moins une partie de percussion (24 ; 66, 68 ; 96, 98, 100), qui est déplacée le long d'un tronçon d'accélération.

6. Appareil selon la revendication 5, **caractérisé en ce que** la partie de percussion (24; 66, 68; 96, 98, 100) est fabriquée à partir d'un matériau présentant un faible poids spécifique, par exemple à partir de matière plastique ou d'un métal léger, par exemple à partir de titane, d'aluminium ou similaire.

7. Appareil selon la revendication 5 ou 6, **caractérisé en ce que** la partie de percussion (24 ; 66, 68 ; 96, 98, 100) présente des évidements, des cavités, des renfoncements ou des ouvertures.

8. Appareil selon l'une quelconque des revendications 5 à 7, **caractérisé en ce que** la partie de percussion (24 ; 66, 68 ; 96, 98, 100) est accélérée à une vitesse d'impact lors de l'impact sur la partie d'applicateur, laquelle est supérieure à 20 m/s.

9. Appareil selon l'une quelconque des revendications 5 à 8, **caractérisé en ce que** les coups, à l'action desquels le côté proximal de la partie d'applicateur peut être soumis de manière répétée, sont provoqués par au moins deux parties de percussion (66, 68 ; 96, 98, 100), qui sont déplacées le long respectivement d'un tronçon d'accélération.

10. Appareil selon la revendication 9, **caractérisé en ce que** les parties de percussion (66, 68) viennent heurter la partie d'applicateur sensiblement de manière simultanée.

11. Appareil selon la revendication 9, **caractérisé en ce que** les parties de percussion (96, 98, 100) viennent heurter les unes après les autres la partie d'applicateur.

12. Appareil selon la revendication 11, **caractérisé en ce que** la partie d'applicateur (94) peut être basculée à partir de son axe médian longitudinal (120), et que les parties de percussion (96, 98, 100) viennent heurter la partie d'applicateur (94) à l'extérieur de l'axe médian longitudinal (120).
